# EUROPEAN PATENT APPLICATION

(11) **EP 3 342 382 A1**
(43) Date of publication of application: **04.07.2018**
(21) Application number: 17150110.9
(22) Date of filing: 03.01.2017
(51) Int. Cl.: A61F 13/08, B32B 5/00, B32B 7/00

(54) **COMPRESSION BANDAGE**

(71) Applicant: 3M Innovative Properties Company, Saint Paul, MN 55133-3427 (US)
(72) Inventor: Hitschmann, Guido, 41470 Neuss (DE); Bongards, Christine, 41516 Grevenbroich (DE); Carter, Daniel J., Saint Paul, Minnesota 55133-3427 (US); Kees, William D., Saint Paul, Minnesota 55133-3427 (US); Rogers, John Joseph, Saint Paul, Minnesota 55119 (US)
(74) Representative: Müller, Bruno

(57) **Abstract**

Compression bandage for use as the only bandage in a single-bandage system for compression therapy, comprising an elastic compression layer and an elastic comfort layer, of which at least 50% is permanently attached to the compression layer.

The compression bandage has a full-stretch elongation (FSE) of between 25% and 60% beyond its unstretched length. Full-stretch elongation is defined herein as the elongation at which the second derivative (130) of a curve (110) showing tensioning force over longitudinal elongation has its global maximum (140).

## Description

This disclosure relates to compression bandage systems for use in therapy of venous leg ulcer and edema, and in particular to single-bandage compression systems, that provide effective therapy in the absence of other bandages.

Two-bandage compression systems are being widely used in compression therapy of venous leg ulcer, where an elastic inner bandage is wrapped around the patient's calf to provide some cushioning and a small amount of compression, and an outer elastic bandage is wrapped around the calf, over the inner bandage, to provide the major part of the compression. A two-bandage system is described, for example, in the European Patent EP 1871316 B1.

Alternatively, single-bandage compression systems are known which have a skin-facing layer and an outer layer. The compression bandage described in the international patent application WO 2009/071894 A1, for example, is described as being usable alone. Single-bandage systems tend to be easier and quicker to apply than two-bandage systems.

When a single-bandage compression system (which is also referred to herein as "single-bandage") is applied around the patient's calf by different persons or by the same person at different times, the elongation at which the bandage is applied generally varies from application to application. For a successful compression therapy, however, it is important that the bandage is applied around the patient's calf every time, i.e. consistently, with a specific tension, which provides a specific resistance to short-term expansion of the calf, e.g. when the patient walks. This resistance is often referred to as "stiffness" of the compression system. Stiffness relates to the force (i.e. force per unit width of material per percent additional elongation, e.g. N/cm/%) required to elongate the bandage further by a small amount.

The specific tensioning force , at which the compression bandage is to be applied, requires a specific elongation of the bandage. As compression bandages are often applied and worn by elderly or weakened patients, both the required force and the required elongation should be within the reach of a weak person, whose arms may only have a limited physical strength and a limited range within which the patient can extend them.

The present disclosure attempts to address these needs. The present disclosure provides a compression bandage, for wrapping around a human calf, for use as an only bandage in a single-bandage system for compression therapy of venous leg ulcer and edema, comprising a) an elastic compression layer having two opposed major surfaces, and b) an elastic comfort layer for providing cushioning between the compression layer and the calf, having a major surface, of which at least 50% is permanently attached to a major surface of the compression layer. The compression bandage is characterized by having a full-stretch elongation of between 25% and 60% beyond its unstretched length, with full-stretch elongation being defined as the elongation at which the second derivative of a curve showing tensioning force over longitudinal elongation has its global maximum.

The provision of a single-bandage having a full-stretch elongation helps to obtain consistent application elongation, because the caregiver or the patient can simply stretch the bandage manually up to the full-stretch elongation and then apply it to the calf with this elongation. The full-stretch elongation is simple to "find", because the bandage requires less force to elongate it further as long as its elongation is below the full-stretch elongation, and requires noticeable more force to elongate it further when the full-stretch elongation has been reached. Once applied at the full-stretch elongation, the bandage provides a noticeable resistance to further stretching, which results in an improved efficiency of the compression therapy.

The full-stretch elongation is not the maximum elongation of the single-bandage according to the present disclosure. A single-bandage according to the present disclosure can be elastically elongated beyond the full-stretch elongation, because it is supposed to provide elastic compression when the patient walks whereby the calf circumference increases, even when the single-bandage was applied at its full-stretch elongation when the patient was sitting and his calf had a smaller circumference.

The full-stretch elongation of a single-bandage according to the present disclosure is in the range between 25% and 60% elongation of the single-bandage beyond its unstretched length. This range allows a weak patient who may not be able to exert high stretch forces with his hands far apart to apply the single-bandage to his own calf. An elongation range of 25% to 60% elongation to reach full-stretch requires less arm range of a patient.

A bandage that is suitable for compression therapy of venous leg ulcer and edema is a bandage that provides a sufficient therapeutic pressure on the calf for that type of therapy, and that can provide a sufficiently high static stiffness index (difference between pressure on the calf in a standing position and pressure in a supine position) of at least 10 mmHg.

An advantage of the compression bandage of the present disclosure is that it combines a cushioning layer and a compression layer in one single-bandage. A "single-bandage compression system" or a "single-bandage system for compression" is a compression system which comprises a single bandage. In such systems no second or other further bandage is present, so that the single-bandage is the only bandage in the compression system. A single-bandage system is thus characterized by the absence of any second or further bandage(s). A single-bandage system for compression therapy of venous leg ulcer and edema is a single-bandage that can provide the required therapeutic compression for compression therapy of venous leg ulcer and edema in the absence of any second bandage. Single-bandages that cannot provide the required therapeutic pressure over an adequate time for compression therapy of venous leg ulcer and edema, e.g. due to their mechanical properties, in the absence of any second bandage are not considered to be single-bandage compression system for compression therapy of venous leg ulcer and edema in the context of this disclosure.

The term "bandage", in the context of this disclosure, refers to an elongate piece of material that can be wound or wrapped longitudinally around the circumference of the patient's calf in multiple turns.

Compression bandages according to the present disclosure may have widths of between 4 centimetres and 25 centimetres in an unstretched state. More preferably, a compression bandage according to the present disclosure has a width of between 7 and 14 centimetres in an unstretched state. These widths have been found to be practical in use and easy to apply in compression therapy of venous leg ulcer and edema.

Independent of their widths, a compression bandage according to the present disclosure may have a thickness of between 0.5 millimetre and 10 millimetres in an unstretched state. Bandages with such thicknesses can provide adequate cushioning, while still being thin enough to be worn inconspicuously under trousers.

An elastic layer, in the context of the present disclosure, refers to a layer which can be elastically deformed in at least one direction parallel to its major surfaces. When elongated, expanded or stretched, an elastic layer strives to return to its unexpanded shape. When stretching an elongate bandage longitudinally, it strives to return to its unstretched length. Elastic deformation is understood herein to be fully reversible after releasing, while plastic deformation is understood to be not reversible or not fully reversible. Plastic deformation occurs, for example, when a bandage is elongated far enough for elements of it to rupture.

A compression bandage according to the present disclosure comprises at least two layers: a compression layer and a comfort layer, permanently attached to the compression layer. The compression layer is elastic. It may be adapted to provide a major part of the therapeutic compression on the calf.

The compression layer may be adapted to be the outermost layer of the compression bandage, furthest from the skin, when the bandage is wrapped around the calf. In compression bandages having a third layer, however, the compression layer may be an inner layer of the compression bandage. In that case, the third layer may form the outermost layer.

The compression layer is elastic, i.e. it may be elastically expanded at least in the long direction of the compression bandage. The compression layer may be adapted to provide a compressive force on a calf after the bandage is wound around the calf in an expanded state. The compression layer has two opposed major surfaces. The compression layer may be an elastic web or an elastic fabric, the web or the fabric having a thickness of, for example, between about 0.1 mm and 5.0 mm.

The compression layer may comprise an elastomeric material. The compression layer may comprise a woven, knitted or a nonwoven web. The web may be a nonwoven fibre web. The woven, knitted or a nonwoven web may comprise a plurality of longitudinally extending elastic yarns.

The woven, knitted or a nonwoven web may be coated or impregnated with a binder or otherwise comprise a binder. The binder may be a polymeric binder. The binder may be adapted such that a portion of its surface adheres to another portion of its surface when the portions are brought into contact. This property of being "capable of adhering to itself" is referred to herein as the binder - or a surface coated with it - being "idio-adhesive". The polymeric binder may be an elastomeric polymeric binder or a non-elastomeric polymeric binder. Suitable elastomeric polymeric binders may comprise natural rubber latex, a synthetic latex, such as homopolymer and copolymer latexes of acrylics, butadienes, styrene/butadiene rubbers, chloroprenes, ethylenes (e.g., vinyl acetate/ethylene), isoprenes, nitriles and urethanes, or mixtures thereof. Examples of suitable polymeric elastomeric binders are disclosed for example in U.S. Patent Nos. 3,575, 782; 4,984,585; and 6,155,424 and in textbooks.

The comfort layer of the compression bandage is elastic, i.e. it may be elastically expanded at least in the long direction of the compression bandage. The comfort layer is typically thicker than the compression layer. It has cushioning properties to level out potential pressure peaks in certain locations on the skin and thereby improves the wearing comfort of the bandage. Too much cushioning, e.g. by a too soft and too thick cushioning layer, however, must be avoided in order to ensure that even small elongations beyond the full-stretch elongation provide considerable resistance and thus a sufficient therapeutic compression and sufficient "stiffness" of the bandage.

Without cushioning, the compression layer could cause excessive pressure on protruding portions of the calf, e.g. next to protruding bones. The comfort layer is suitable for providing cushioning between the compression layer and the calf. The comfort layer may be an innermost layer of the compression bandage, when the bandage is applied around the calf. In other words, the comfort layer may be a skin-facing comfort layer. Generally, the comfort layer may be arranged and adapted for contacting the skin when the compression bandage is wrapped around the calf. In this case, the comfort layer can also be referred to as a skin-contacting comfort layer. Where the comfort layer is a foam layer, it may also be referred to as a skin-contacting foam layer.

Alternatively, the comfort layer may be an inner layer of the compression bandage. This may be the case in compression bandages according to this disclosure which comprise a third layer which is the skin-facing and skin-contacting layer. Where the compression bandage comprises a third layer, the comfort layer may be arranged between the compression layer and the third layer. Generally, in order to provide cushioning, the comfort layer is arranged anywhere between the compression layer and the skin of the calf, when the bandage is wrapped around the calf.

The comfort layer may comprise a foam, or it may consist of a foam. The comfort layer may comprise a foam for providing cushioning. The foam may be a polymeric foam. In certain embodiments of the compression bandage according to the present disclosure, the comfort layer may thus be a foam layer. Suitable foams can provide adequate cushioning, while also being able to absorb a certain amount of liquid such as wound exudate. The foam of a skin-contacting comfort layer of a bandage as disclosed herein may provide, besides cushioning, water vapour transmission and/or breathability. These increase the patient's comfort when wearing the bandage.

It has been found particularly advantageous if the comfort layer is a skin-contacting foam layer. In use, the exposed face of the skin-contacting foam layer exhibits a particularly desirable and effective fastening of the compression bandage onto the skin of the patient, which minimizes of tendency of the compression bandage to slip after application and during use.

At least a portion of a major surface of the comfort layer is permanently attached to a major surface of the compression layer. Specifically, the comfort layer has two opposed major surfaces, of which one is oriented towards the skin, and the opposed surface is oriented towards the compression layer. Of the major surface oriented towards the compression layer, at least 30%, preferably at least 50% is permanently attached to a major surface of the compression layer. In certain embodiments, between 95% and 100% of the major surface of the comfort layer is permanently attached to a major surface of the compression layer. Preferably, however, the entire major surface (i.e. 100%) of the major surface of the comfort layer is permanently attached to a major surface the compression layer. Where a considerable percentage of the comfort layer major surface is permanently attached to a major surface of the compression layer, there is generally less risk of improper application of the two-layer compression bandage around the calf and less risk of damage to the bandage due to loose portions of the comfort layer being caught when handling the bandage before, during and after application around the calf.

In one aspect of the present disclosure, the comfort layer may be permanently attached to the compression layer directly or indirectly. In certain embodiments, the major surface (or a portion of the major surface) of the comfort layer is permanently attached to a major surface of the compression layer directly, i.e. without intermediate layers. In certain embodiments, the at least 50% of the major surface of the comfort layer is directly permanently attached to a major surface of the compression layer. In certain other embodiments, the at least 50% of the major surface of the comfort layer is permanently attached to a major surface of the compression layer indirectly, e.g. via one or more intermediate layers, or directly, i.e. without intermediate layers. In these embodiments, the comfort layer and the compression layer are adjacent layers of the compression bandage.

Where the comfort layer is permanently attached directly to the compression layer, the attachment may be, for example, an adhesive attachment or an attachment via surface welding, i.e. melting the surfaces and bringing them into contact before resolidifying. Methods like needle-punching or spot welding provide a much smaller attachment area between the major surface of the compression layer and the major surface of the comfort layer, and may increase the risk of damage when the bandage is stretched.

A permanent attachment, in this disclosure, refers to an attachment that is not intended to be releasable or that is not releasable, e.g. during normal use or handling of the bandage. A portion of the major surface of the comfort layer is considered permanently attached to the major surface of the compression layer if the comfort layer cannot be released from the compression layer in normal use or handling of the compression bandage, or with manual forces which are common when handling compression bandages. A comfort layer that can be released or separated from the compression layer using, for example, excessive force, using tools or using chemicals, but not in normal use and handling or using common manual forces is still considered permanently attached to the compression layer herein.

The term "elongation", as used herein, refers to longitudinal elongation (i.e. elongation in the long direction) beyond the unstretched length. An elongate bandage of 10 cm width and 100 cm length before stretching may be stretched in its long direction to an elongation of, for example, 35%. At that elongation of 35%, the bandage has a length, in its long direction, of 135 cm.

Longitudinal elongation and corresponding tensioning forces can be determined in tensile testing machines. In tensioning-force-over-longitudinal-elongation diagrams, obtainable using such machines, the elongation in percent is the abscissa, while the resulting tensioning force in Newton (N) or Newton per cm of nominal sample width (N/cm) is the ordinate.

A compression bandage according to this disclosure is stretched in its long direction to a certain elongation beyond its unstretched length before it is applied longitudinally around a patient's calf in turns, so that it is wrapped around the calf under tension. After wrapping the bandage around the calf, friction between the bandage and the skin or between a first portion and a second portion of the bandage prevents the bandage from returning into its unstretched state, so that the bandage keeps its tension. The tension causes the bandage to exert therapeutic pressure on the calf.

It is also important in single-bandage compression systems that the bandage is applied around the patient's calf at a suitable tension for effective support of the so-called muscle pump. When an elastic bandage is manually applied, the tension can vary between a low tension and a high tension, depending on the amount of force that the patient or the caregiver applies to stretch the bandage before beginning to wind it around the calf. Certain bandages can be stretched up to a specific elongation with less force, but require considerable more force to stretch them beyond that elongation. The elongation, from which onward considerable higher force is required for additional elongation is often referred to as the "full-stretch elongation".

In a tensioning-force-over-elongation diagram, the part of the curve which reflects the "less force required" regime (i.e. elongations below the full-stretch elongation) has a lower slope than the part above the full-stretch elongation, which reflects the "considerable more force required" regime. In the transition zone around the full-stretch elongation, the slope of the curve changes from low to high, with increasing elongation. This change in slope of the curve is visible in the first derivative of the curve as a step, and in its second derivative as a maximum.

The full-stretch elongation of an elastic bandage material may thus be defined as the elongation at which the second derivative of the curve showing tensioning force of the material over its longitudinal elongation has a global maximum.

The full-stretch elongation of a bandage of a certain width can be systematically determined by stretching a 10 cm wide segment of the bandage longitudinally, in a tensile testing machine at room temperature with a jaw distance of 200 mm at an elongation speed of 500 mm/minute, and by recording the tensioning force for each elongation. This measurement can be done according to the standard test method laid down in DIN EN 13934-1, i.e. with a pre-force of 0.1 N, a sample length of 250 mm and a jaw distance of 200 mm, except that the elongation speed is not 100 mm/minute, but 500 mm/minute. The data pairs of (elongation in % of unstretched length; tensioning force in Newton per cm of width of the bandage) or "(%E; Ft)" can be plotted in an x-y diagram to form a curve. In that diagram, %E is the abscissa, and Ft is the ordinate. Suitable fitting algorithms may be used to obtain an analytic expression for this curve in the relevant elongation regime. Forming the first and second derivative of the curve and the global maximum of the second derivative can then be done analytically. Alternatively, data pairs can be interpolated and methods using discrete difference quotients can be used to obtain discrete values for the first and the second "derivative" and for identifying the elongation position of the global maximum of the second derivative. Other known methods may be used alternatively.

Depending on the method used to determine the elongation at which the second derivative has its global maximum, the full-stretch elongation may slightly vary. A typical variation is estimated to be less than 10%, probably rather 3-5% of the full-stretch elongation value found using one of the possible methods. The global maximum is normally formed by a series of three or more data pairs, and is therefore easy to distinguish from isolated sharp peaks caused by one or two data pairs, which are normally the effect of artefacts in the measured data.

The full-stretch elongation of the compression bandage may therefore be alternatively defined as the elongation at which lies the maximum of an at least 3% full-width-half-maximum-wide peak of the second derivative of a curve showing tensioning force over percentage longitudinal elongation. In a yet further alternative, the full-stretch elongation of the compression bandage may be defined as the elongation at which lies the maximum of an at least 2% full-width-half-maximum-wide peak of the second derivative of a curve showing tensioning force over percentage longitudinal elongation. In these definitions, the 2% or 3% widths refers to the percentage values on the abscissa axis (x axis). These definitions help exclude artefacts which mostly generate very narrow peaks, narrower than 2% and 3% respectively. As per the commonly known definition of full-width-half-maximum (FWHM), the half-maximum value refers to the maximum force at the tip of the peak, divided by two.

The tensioning-force-over-elongation curve may be somehow different for different widths of the same bandage material being tested. This is believed to be due to a degree of lateral contraction when the bandage is stretched longitudinally, which absorbs some of the tensioning force. As a result, the corresponding longitudinal tensioning force per cm of width at the full-stretch elongation is often higher for narrower bandages than for wider bandages of identical material. In order to obtain comparable measurement data, a 10 cm wide segment of any bandages to be tested should be subjected to stretching in the tensile testing machine. Generally, for comparing tensile data of different bandages, the test samples should have the same width.

A bandage in accordance with the present disclosure may be obtained, for example, by attaching an existing compression layer and an existing comfort layer to each other, e.g. by lamination. Alternatively, individual layers, obtained by delaminating existing multilayer bandages, may be attached to each other at their major surfaces to form a compression bandage of a single-bandage system as described herein. For example, an individual layer of compression bandages available under the names 3M™ Coban™ 2, 3M™ Coban™ 2 Lite, 3M™ Coban™ LF or 3M™ Coban™ LF Hand tear, available form 3M Company, may be usable as the compression layer of a bandage according to this disclosure.

Commercially available foam materials may be used to form the comfort layer of a compression bandage according to the present disclosure. Alternatively, the foam layer of an existing multilayer bandage may be delaminated (i.e. separated) from the remaining layers of the bandage and may be attached to a compression layer at their major surfaces to form a compression bandage of a single-bandage system according to the present disclosure. For example, the foam layer of an existing compression bandage available under the name 3M™ Coban™ 2 Lite, available form 3M Company, may be usable as the comfort layer of a bandage according to this disclosure.

Alternative methods to obtain potentially suitable compression layers that can be attached to suitably selected comfort layers to form bandages according to the present disclosure are described, for example, in U.S. Patents No. 3,575,782, 4,984,584, and 7,135,213 B2.

In order to obtain a bandage having the desired full-stretch elongation, the selected compression layer material may be pre-stretched to a certain degree when attaching a selected unstretched comfort layer material to it. For the same purpose, the selected comfort layer material may be pre-stretched to a certain degree when attaching a selected unstretched compression layer material to it. For the same purpose the selected comfort layer material may be pre-stretched to a certain degree when attaching a suitably selected compression layer material, pre-stretched to the same degree as the comfort layer material or to a different degree, to it to form a single-bandage according to the present disclosure.

A suitably selected compression layer and a suitably selected comfort layer, once attached to each other, may form a single-bandage according to the present disclosure, that has suitable mechanical properties, such as a desired full-stretch elongation.

In certain preferred embodiments of the bandage according to the present disclosure, the bandage has a full-stretch elongation of between 35% and 60% beyond its unstretched length.

Having a full-stretch elongation further from the unstretched length is beneficial for the following reason: A human calf has a generally conical shape with a smaller circumference closer to the ankle and a larger diameter closer to the knee. When a compression bandage of a certain width is wrapped around the calf in turns, the upper edge of the bandage (the edge closer to the knee) will normally be kept at the full-stretch elongation by friction, once applied on the calf. At the lower edge (closer to the ankle), however, the smaller circumference of the calf does not provide resistance to the bandage against contraction. The lower edge of the bandage will thus contract down to either a length where it meets the circumference of the calf, or alternatively to its unstretched length, whatever length it attains first when contracting. If its full-stretch elongation is only 10% beyond its unstretched length, the lower edge of the bandage can only contract by these 10% before it reaches its unstretched length, which may not be sufficient to meet the circumference of the calf. In that case the lower edge of the bandage will not overlie the calf smoothly and conform to its surface, but rather form a loose portion, away from the skin. Such a loose portion may be considered aesthetically unpleasing and also poses a risk of generating a pressure peak on the skin when its position happens to be under the upper edge of the subsequent turn of the bandage. Similar problems can occur when a turn of the bandage is wrapped over a protrusion, e.g. the patella or a deformation or a wound dressing, where one width section of the bandage is elongated more than another width section, but where the other section is still supposed to conform closely to the skin. In general, the effects described here are more prominent with bandages of greater width than with narrower bandages.

Depending on their widths, compression bandages where the full-stretch elongation is at higher elongations, such as between 35% and 60% of the unstretched length, are less likely to form loose lower edges, because the lower edge of such a bandage can contract by 35% or even 60% (of its unstretched length) before it reaches its unstretched length. This larger potential contraction length may be sufficient to meet the circumference of the calf and to avoid loose portions, not in contact with the skin.

In an aspect of the present disclosure, the compression bandage provides specific tensioning forces at its full-stretch elongation. In certain embodiments, the compression bandage provides, at its full stretch elongation, a tensioning force in longitudinal direction of at least 0.5 Newton per centimetre width of the compression bandage. Width of the bandage here refers to its width in its unstretched state, its "nominal width". Bandages having this minimum tensioning force at their full-stretch elongation can still provide adequate therapeutic compression for an effective therapy, provided that the bandage is applied such that subsequent turns of the bandage overlap to a sufficient degree. For bandages providing, at full-stretch elongation, a relatively low tensioning force in longitudinal direction of about 0.5 to 1.0 Newton per centimetre nominal width, overlaps of between 50% width and 75% width between subsequent turns may be required to obtain adequate therapeutic compression. Also, bandages providing this low tensioning force at their full-stretch elongation are less likely to slip when worn on the calf, because the skin-facing layer, e.g. the foam of the comfort layer, are pressed against the skin with a certain pressure.

In certain embodiments, the compression bandage provides, at its full stretch elongation, a tensioning force in longitudinal direction of not more than 4.0 Newton per centimetre nominal width at its full stretch elongation. Tensioning forces below 4.0 N/cm at full-stretch elongation provide a safe level of therapeutic compression and are less likely to compress the calf too much, which could result in blocking of some veins. For bandages providing, at their respective full-stretch elongation, a relatively high tensioning force in longitudinal direction of about 3.0 to 4.0 Newton per centimetre nominal width, low overlaps of between 5% width and 20% width between subsequent turns may be required to avoid excessive compression.

In certain embodiments, the compression bandage provides, at its full stretch elongation, a tensioning force in longitudinal direction of between 0.5 Newton per centimetre nominal width of the compression bandage and 4.0 Newton per centimetre nominal width. These bandages combine the advantages of provision of therapeutically effective pressure, reduced slippage and of safe degree of compression.

The compression bandages as disclosed herein can be stretched elastically beyond their full-stretch elongation, although they become "stiffer" at elongations beyond the full-stretch elongation, i.e. they require a higher tensioning force to stretch them by another 1% elongation beyond their respective full-stretch elongation. The full-stretch elongation and a certain further elastic elongation, beyond the full-stretch elongation, can thus be obtained by stretching the bandage elastically, such that it returns to its unstretched length after the tensioning force is removed. However, when stretching the bandage further and further beyond its full-stretch elongation, from a certain elongation onwards the bandage will be plastically deformed, i.e. it is deformed irreversibly and loses its ability to return elastically to its unstretched length. When stretching even further, the bandage will ultimately rupture and be destroyed.

A bandage according to the present disclosure may be elastically stretchable by a total tensioning force of at least 6.0 Newton per centimetre nominal bandage width. It may be elastically stretchable by a total tensioning force of at least 6.0 Newton per centimetre nominal bandage width without being plastically deformed or damaged or essentially without being plastically deformed or damaged.

Bandages that can withstand a tensioning force of at least 6.0 Newton per centimetre nominal width are likely to survive occasional excessive stretching, e.g. by unusual muscle movements or when being handled by unexperienced patients, and may thus have a longer lifetime and greater cost efficiency.

Bandages according to the present disclosure are elastically stretchable beyond their respective full stretch elongation. Above the full-stretch elongation they require considerably more tensioning force to stretch them by another percent elongation than they require at elongations below the full-stretch elongation. This behaviour provides for a "stiff' bandage, which resists strongly to further tension after being applied around the calf at full-stretch elongation, and thereby helps obtain a high static stiffness index. The static stiffness index is usually defined as the difference, measured by a sub-bandage pressure sensor in mmHg, between sub-bandage pressure when the patient stands and sub-bandage pressure when the patient lies.

The desirable stiff behaviour of the bandage above its full-stretch elongation is reflected in the shape of the curve showing tensioning force per nominal bandage width over percent longitudinal elongation: at elongations above the full-stretch elongation, the slope of the curve is higher than below the full-stretch elongation.

In compression bandages as described herein, the slope of the curve showing tensioning force over longitudinal elongation of the bandage between the full-stretch elongation and 1.1 times the full stretch elongation may thus be at least 0.25 Newton per centimetre nominal bandage width per percent elongation. The slope of the curve may be at least 0.25 Newton per centimetre nominal bandage width per percent elongation in at least one elongation between the full-stretch elongation and 1.1 times the full stretch elongation. Herein, the full-stretch elongation may be the full-stretch elongation expressed in percent elongation beyond unstretched length.

Bandages according to the present disclosure provide tactile feedback as to at which elongation the bandage is to be applied around the calf, namely the increased resistance to stretching when reaching the optimum (i.e. full-stretch) application elongation. In an aspect of this disclosure, for redundancy, an additional feedback mechanism may be implemented. For example, visible indicia on the bandage may indicate when the optimum application elongation has been reached, namely the full-stretch elongation. In certain embodiments, the compression bandage further comprises visible indicia, arranged such as to be visible when the bandage is being applied, indicating a degree of stretching of the bandage. In certain embodiments, the compression bandage further comprises visible indicia, arranged such as to be visible when the bandage is being applied, indicating the attainment of the full-stretch elongation of the bandage. In some of these embodiments, the visible indicia may comprise a printed element, which is ellipsoid-shaped at elongations below the full-stretch elongation, and which has a circular shape at the full-stretch elongation. The shape of the ellipsoid indicates a degree of stretching. The closer the elongation is to the full-stretch elongation, the more the ellipsoid takes the shape of a circle.

A compression bandage as described herein may be useful in compression therapy of venous leg ulcer on the calf. To support the compression therapy, it may be considered useful to apply additional compression, albeit to a lower degree, on the foot. The bandage may therefore be combined, in a kit, with a sock that can apply a low degree of compression on the foot. Alternatively, or on top, the bandage may be combined, in a kit, with a compression stocking that can apply a low degree of compression on the foot and a minimal compression on the calf. Such a stocking could be worn over the calf compression bandage in order to hide the presence or the appearance of the calf compression bandage.

Therefore, in a yet further aspect, the present disclosure provides a kit of parts, comprising a compression bandage as described herein, and either
a) a compression stocking, adapted to provide therapeutic compression on the foot of between 5 mmHg and 40 mmHg and to provide therapeutic compression above the ankle of less than 10 mmHg, or
b) a sock, adapted to provide therapeutic compression on the foot of between 5 mmHg and 40 mmHg. The mmHg pressure values refer to sub-bandage pressures obtained using a pressure sensor positioned between the bandage and the calf.

The invention will now be described in more detail with reference to the following Figures exemplifying particular embodiments of the invention, wherein like elements are indicated by the same reference numeral:
- Fig. 1: Perspective sectional view of a compression bandage according to the present disclosure; and
- Fig. 2: Tensioning-force-over-elongation diagram of a compression bandage according to the present disclosure, and its first and second derivative.

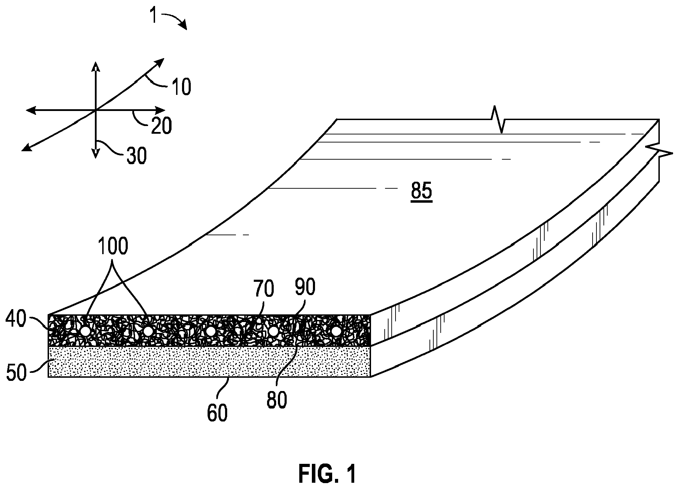

In the perspective sectional view of **Figure 1****,** a compression bandage 1 according to the present disclosure is illustrated. The bandage 1 is a single-bandage, i.e. it is the only bandage in a single-bandage system for compression therapy of venous leg ulcer or edema. The bandage 1 has an elongate shape: it extends longitudinally in length directions indicated by arrow 10, while arrow 20 indicates width directions and arrow indicates 30 thickness directions. The compression bandage 1 can be wrapped around a human calf in its longitudinal direction 10 in several turns. The bandage 1 has a width 20 of 14 centimetres and a thickness 30 of 2.5 millimetres.

The bandage 1 is elastically stretchable in longitudinal directions 10. It comprises two layers: a compression layer 40 and a comfort layer 50.

The comfort layer 50 is the inner, skin-facing layer of the compression bandage 1, when the bandage 1 is applied around the calf. Its exposed surface 60 contacts the patient's skin when the bandage 1 is applied. The comfort layer 50 is elastically stretchable in longitudinal directions 10. It consists of a soft, conformable polyurethane foam and provides cushioning between the compression layer 40 and the calf.

The compression layer 40 is also elastically stretchable in longitudinal directions 10. The compression layer 40 has two parallel opposed major surfaces: an upper, exposed major surface 85 and a lower, "inner" major surface 80.

The compression layer 40 comprises a nonwoven web 90 of cellulose acetate fibres and a plurality of elastic yarns 100, embedded in the nonwoven web 90 and extending in longitudinal directions 10. The nonwoven web 90 and the elastic yarns 100 are coated with an acrylic polymeric binder. The binder is idio-adhesive, i.e. it can adhere to itself, and it can also be made to adhere permanently to the foam of the comfort layer 50 by laminating the compression layer 40 and the comfort layer 50 together, thereby providing an intimate contact between the foam of the comfort layer 50 and the binder-coated non-woven web 90.

The non-exposed, i.e. "inner" major surface 70 of the comfort layer 50, located opposed to the exposed major surface 60, is attached directly and completely to the lower, non-exposed, "inner" major surface 80 of the compression layer 40.

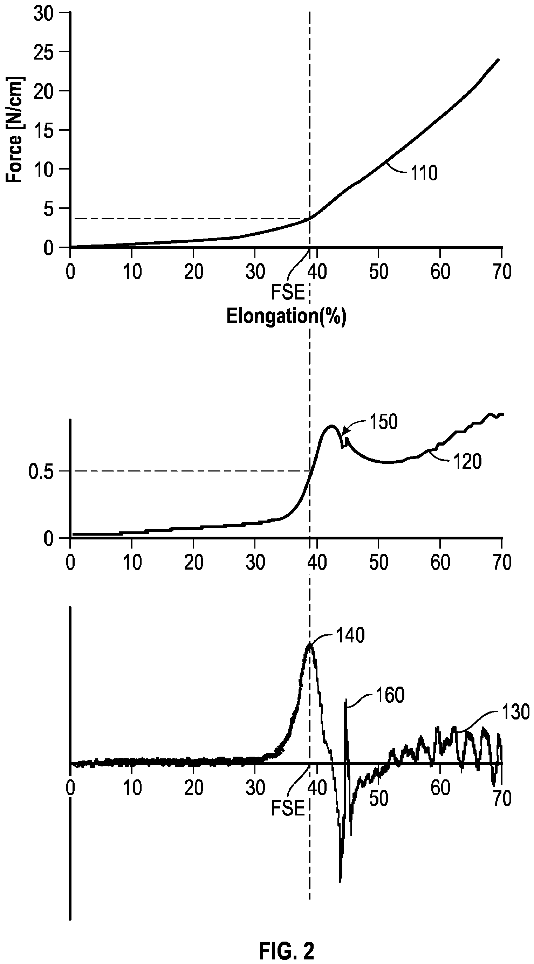

The elastic properties of the compression layer 40 and of the comfort layer 50 attached to the compression layer 40 provide suitable elastic properties to the entire compression bandage 1. These properties can be determined on a tensile testing machine. **Figure 2** shows, in a diagram of tensioning force over elongation, a result of such determination of a 10 cm wide section of the bandage 1 shown in Figure 1.

The curve 110 in the top diagram in Figure 2 shows the tensioning force in Newton per cm width of the section of the bandage 1 (ordinate) over percent elongation beyond the unstretched length of the section of the bandage 1 (abscissa). The measurement was taken at an initial jaw distance of the testing machine of 200 mm, corresponding to the length 10 of the section of bandage 1 tested. The measurement was run at an elongation speed of 500 mm/minute, by which the one end of the section of the bandage 1 was pulled away from the opposite other end in the testing machine.

The curve 110 shows a slower increase of tensioning force from the unstretched state of the bandage section (0% elongation) up to about 38%-40% elongation. The small slope of the curve 110 in this "softer" regime reflects the property of the bandage 1 to allow elastic elongation with relatively little force up to about 38%-40% elongation.

In the "harder" regime beyond 40% elongation, the steeper slope of the curve 110 reflects the property of the bandage 1 to require noticeable more force than in the lower-elongation regime to elongate it further.

The transition between the softer and the harder regime occurs around the full-stretch elongation. In the context of this disclosure, the full-stretch elongation is defined as that elongation at which the second derivative of the curve showing tensioning force over longitudinal elongation has its global maximum. The middle diagram of Figure 2 shows the curve of the first derivative 120 of the curve 110 versus percent elongation. The bottom diagram of Figure 2 shows the curve of the second derivative 130 of the curve 110, in arbitrary units, versus percent elongation.

The curve 130 of the second derivative shows the first clear maximum 140, which is at the same time the global maximum 140, located at an elongation of about 39% elongation. The full-stretch elongation of the section of the bandage 1 under test is thus the elongation of 39% beyond the unstretched length of the bandage. This specific elongation is marked with "FSE" in Figure 2.

It may be noted that an artefact 150 appears in the curve 120 of the first derivative at about 45% elongation. This artefact appears as a secondary peak 160 in the second derivative 130, with its maximum lower than the global maximum 140. However, even if the peak of the artefact 160 were higher than the global maximum 140, it would not qualify as the global maximum of the curve 130 of the second derivative because it clearly originates from an artefact in the measurement data. In order to avoid such artefacts, the tensioning force curve 110 might be smoothed using known algorithms, while keeping the overall shape of the curve 110.

The full-stretch elongation of the bandage 1 now being identified as 39%, the tensioning force curve 110 allows to identify the tensioning force which is required to stretch the bandage 1 to its full-stretch elongation. The full-stretch elongation abscissa value of 39% corresponds to an ordinate value of about 3 N/cm, as can be seen in the top diagram of Figure 2. A force of about 3 N/cm is thus required to stretch the bandage 1 to its full-stretch elongation of 39%. Stretching the bandage 1 by another 5% elongation to a total elongation of 44% requires a considerable additional force, namely about 2.5 N/cm, as can be determined by multiplying the value of the first derivative 120 at the 39% full-stretch elongation (about 0.5 N/cm/%) with the 5% additional elongation. The total tensioning force per cm bandage width required to stretch the bandage 1 to 44% elongation is thus about 5.5 N/cm.

## Claims

1. Compression bandage (1),
for wrapping around a human calf,
for use as an only bandage in a single-bandage system for compression therapy of venous leg ulcer and edema,
comprising
a) an elastic compression layer (40) having two opposed major surfaces (80, 85), and
b) an elastic comfort layer (50) for providing cushioning between the compression layer and the calf, having a major surface (70), of which at least 50% is permanently attached to a major surface (80) of the compression layer, **characterized by** the compression bandage having a full-stretch elongation (FSE) of between 25% and 60% beyond its unstretched length,
with full-stretch elongation being defined as the elongation at which the second derivative (130) of a curve (110) showing tensioning force over longitudinal elongation has its global maximum (140).

2. Compression bandage according to claim 1, having a full-stretch elongation of between 35% and 60% beyond its unstretched length.

3. Compression bandage according to claim 1 or claim 2, providing, at its full stretch elongation, a tensioning force in longitudinal direction (10) of between 0.5 Newton per centimetre nominal width (20) of the compression bandage and 4.0 Newton per centimetre nominal width.

4. Compression bandage according to any one of the preceding claims, wherein the comfort layer (50) comprises a foam.

5. Compression bandage according to any one of the preceding claims, wherein the comfort layer (50) is arranged and adapted for contacting the skin when the compression bandage is wrapped around the calf.

6. Compression bandage according to any one of the preceding claims, wherein between 95% and 100% of the major surface (70) of the comfort layer is permanently attached to a major surface (80) of the compression layer.

7. Compression bandage according to any one of the preceding claims, wherein the at least 50% of the major surface (70) of the comfort layer is directly permanently attached to a major surface (80) of the compression layer.

8. Compression bandage according to any one of claims 1 to 6, wherein the at least 50% of the major surface (70) of the comfort layer is indirectly permanently attached to a major surface (80) of the compression layer, such as via one or more intermediate layer(s).

9. Compression bandage according to any one of the preceding claims, being elastically stretchable by a total tensioning force of at least 6.0 Newton per centimetre nominal bandage width (20).

10. Compression bandage according to any one of the preceding claims, wherein the compression layer (40) comprises a nonwoven web.

11. Compression bandage according to any one of the preceding claims, wherein the slope of the curve (110) showing tensioning force over longitudinal elongation of the bandage (1) between the full-stretch elongation (FSE) and 1.1 times the full stretch elongation is at least 0.25 Newton per cm bandage width per percent elongation.

12. Compression bandage according to any one of the preceding claims, further comprising visible indicia, arranged such as to be visible when the bandage is being applied, indicating the attainment of the full-stretch elongation (FSE) of the bandage.

13. Compression bandage according to any one of the preceding claims, wherein the bandage has a width (20) of between 7 and 14 centimetres in an unstretched state.

14. Compression bandage according to any one of the preceding claims, wherein the bandage has a thickness (30) of between 0.5 mm and 10 mm in an unstretched state.

15. Kit of parts, comprising a compression bandage (1) according to any one of the preceding claims, and either
a) a compression stocking, adapted to provide therapeutic compression on the foot of between 5 mmHg and 40 mmHg and to provide therapeutic compression above the ankle of less than 10 mmHg, or
b) a sock, adapted to provide therapeutic compression on the foot of between 5 mmHg and 40 mmHg.
